# EUROPEAN PATENT SPECIFICATION

(11) **EP 3 398 536 B1**
(45) Date of publication and mention of the grant of the patent: **29.12.2021**
(21) Application number: 16880454.0
(22) Date of filing: 20.06.2016
(51) Int. Cl.: A61B 17/12, A61B 17/00

(54) **LEFT ATRIAL APPENDAGE OCCLUDER**
VERSCHLUSS FÜR LINKES HERZOHR
DISPOSITIF D'OCCLUSION D'APPENDICE AURICULAIRE GAUCHE

(30) Priority: 31.12.2015 CN 201511030594
(43) Date of publication of application: 07.11.2018
(73) Proprietor: Lifetech Scientific (Shenzhen) Co., Ltd., Shenzhen, Guangdong 518057 (CN)
(72) Inventor: LI, Anning, Shenzhen Guangdong 518057 (CN)
(74) Representative: Prinz & Partner mbB
(86) International application number: PCT/CN2016/086378
(87) International publication number: WO 2017/113646

(56) References cited:
- CN-A- 102 805 654
- CN-A- 103 598 902
- CN-A- 104 287 804
- CN-A- 104 287 804
- CN-A- 104 352 261
- CN-A- 104 856 741
- CN-A- 104 856 741
- CN-A- 104 958 087
- CN-U- 202 143 640
- CN-U- 203 634 235
- CN-U- 204 147 141
- US-A1- 2013 218 193
- US-A1- 2015 005 810

## Description

The present disclosure relates to a medical device, and more particularly relates to a left atrial appendage occluder.

At present, an occluder may be put into a left atrial appendage through a catheter intervention method to prevent a thrombus formed in the left atrial appendage due to atrial fibrillation and avoid apoplexy caused by a fact that the thrombus goes up to a brain, or prevent systematic embolism caused by a fact that the thrombus reaches other portions of a body through the body's blood circulation system. Such left atrial appendage occluders substantially include an integrated occluder and a split occluder according to their structures. For example, the split occluder generally includes a fixing component and a sealing component which are connected with each other; the fixing component is disposed in a cavity of the left atrial appendage to fix the whole occluder; and the sealing component seals an opening portion of the left atrial appendage to prevent blood flow from flowing into the cavity of the left atrial appendage.

For the split occluder of this type, the fixing component is generally arranged in the cavity of the left atrial appendage by means of an anchor, and the anchor punctures the wall of the left atrial appendage to fix the fixing component in the cavity of the left atrial appendage. To reduce a risk that the occluder falls off, the fixing component is generally arranged at a deeper part of the cavity of the left atrial appendage. However, the deeper part of the cavity of the left atrial appendage is also a thinner part of the wall of the left atrial appendage, so that the fixing component will easily pierce the wall of the left atrial appendage, which will cause pericardial effusion, pericardial tamponade, and other adverse consequences.

CN 104287804 A discloses a LAA occluder having a sealing plate, a fixing frame and a connection member therebetween. Wire loops extend radially from a proximal end connecter of the fixing frame then bend toward the distal direction. In a middle section of the fixing frame each wire loop interconnects with two support rods. The two support rods continue in distal direction to form an open-ended fixing frame.

### Summary

In view of the above-mentioned problems, it is necessary to provide a left atrial appendage occluder, which can not only guarantee a stable fixing effect but also reduce the risk of piercing the wall of a left atrial appendage.

According to the present invention, a left atrial appendage occluder is provided as defined in claim 1. The occluder includes a sealing plate, a fixing frame located on one side of the distal end of the sealing plate, and a connection member for connecting the sealing plate with the fixing frame, the fixing frame includes a frame structure; the frame structure includes a proximal side surface and a supporting circumferential surface which is connected with the proximal side surface, and extends from the proximal side surface to the distal end; and the connection member is connected with the proximal side surface.

According to the invention, the radial deformability of the sealing plate is greater than that of the fixing frame, and/or the axial deformability of the sealing plate is greater than that of the fixing frame.

In one embodiment, under an action of a same radial force, a radial length variation of the sealing plate is greater than that of the fixing frame; or under an action of a same radial force, a radial length change rate of the sealing plate is greater than that of the fixing frame; or under the action of the same axial force, a displacement of the sealing plate along an axial force direction is greater than that of the fixing frame along the axial force direction.

In one embodiment, in a naturally unfolded state of the left atrial appendage occluder, the proximal side surface is basically parallel to the sealing plate; the supporting circumferential surface is similar to a columnar surface; a proximal opening of the supporting circumferential surface is connected with the proximal side surface; and a distal opening of the supporting circumferential surface is opened in an impending manner.

In one embodiment, the frame structure includes a proximal end and a plurality of elastic supporting rods; ends of elastic supporting rods are connected with the proximal end in a gathering manner, and the other ends of the elastic supporting rods extend out from the proximal end in a radial manner along a radial direction to form the proximal side surface, and then bend, and axially extend towards the distal end to form the supporting circumferential surface; or,
at least one of the proximal side surface and the supporting circumferential surface includes a plurality of grids formed by the elastic supporting rods mutually connected in an encircling manner.

In one embodiment, the left atrial appendage occluder further includes at least one anchor bar arranged on the frame structure and facing to the sealing plate.

In one embodiment, the fixing frame further includes a film which is arranged on the frame structure and at least covers the supporting circumferential surface.

In one embodiment, the fixing frame further includes at least one anchor bar which is arranged on the supporting circumferential surface and faces to the sealing plate, and the at least one anchor bar penetrates through the film.

In one embodiment, the connection member is a flexible connection member or an elastic connection member.

In one embodiment, the connection member includes a proximal connecting end, a distal connecting end and a connecting body connected between the proximal connecting end and the distal connecting end; the proximal connecting end is connected with the sealing plate; the distal connecting end is connected with the fixing frame; the distal connecting end includes a ball socket; and the distal end of the connecting body includes a ball head matched with the ball socket.

In one embodiment, the connecting body is of an elastic or flexible rod, or a spring structure, or a woven structure formed by a plurality of elastic filaments.

In one embodiment, the sealing plate is a double-layer filament woven structure.

In one embodiment, wherein the supporting rods form an approximately spherical space, each of the supporting rods comprises a proximal arc section, a distal arc section, and a middle arc section connected between the proximal arc section and the distal arc section, and the proximal arc section is connected with the proximal end;
the proximal arc section protrudes towards the distal end of the fixing frame;
the middle arc section protrudes away from the distal end of the fixing frame;
the proximal arc section and the middle arc section form an approximately S-shaped curve; and
the distal arc section protrudes away from a center of the approximately spherical space.

In one embodiment, the fixing frame further comprises a branch connected with two adjacent supporting rods, connection nodes between the branches and the supporting rods are located in the middle portions and/or the distal ends of the distal arc sections.

In one embodiment, two branches are connected between two adjacent supporting rods; and
the two branches are inclined relative to the supporting rods, and extending directions of the two branches are roughly parallel, wherein one branch is connected between the distal arc sections of two adjacent supporting rods, and one end of the other branch is connected to the middle portion of the distal arc section of one supporting rod, and the other end of the other branch is connected to the distal end of the distal arc section.

By optimizing the structure of the fixing frame of the left atrial appendage occluder, the proximal ends of the supporting rods of the fixing frame are gathered, and are fixed by a fixing connector to realize a closed state, and the distal ends of the supporting rods are separated from one another to realize an open state, so that a sufficient supporting force of the fixing frame may be guaranteed, and each supporting rod has certain relative independence, and it may maintain relatively good fitness with a cavity wall of a left atrial appendage to enhance a stable performance of the fixing frame in the left atrial appendage. With the above feature, the fixing frame may be also stably fixed in the left atrial appendage without anchor bars; because of the smooth surface of the fixing frame, the fixing frame will not pierce the cavity wall of the left atrial appendage even if the fixing frame is arranged at a relatively deep position (the cavity wall here is relatively thin) on the left atrial appendage.

### Brief Description of the Drawings

Figure 1 is a schematic diagram of a state of a left atrial appendage occluder provided by one embodiment of the present invention after the left atrial appendage occluder is implanted;
Figure 2 is a schematic diagram of a left atrial appendage occluder provided by one embodiment of the present invention;
Figure 3 is a schematic diagram of a sealing plate of the left atrial appendage occluder in Figure 2;
Figure 4 is a schematic diagram of a connection member of the left atrial appendage occluder in Figure 2;
Figure 5 is a section view of the connection member in Figure 4;
Figure 6 is a schematic diagram of one part of the connection member in Figure 4;
Figure 7 is a schematic diagram of the other part of the connection member in Figure 4;
Figure 8 is a schematic diagram of a fixing frame of the left atrial appendage occluder in Figure 2;
Figure 9 is a schematic diagram of a fixing frame of a left atrial appendage occluder provided by another embodiment of the present invention;
Figure 10 is a schematic diagram of a fixing frame of a left atrial appendage occluder provided by another embodiment of the present disclosure;
Figure 11 is a schematic diagram of a test method of the radial deformability of the fixing frame of the left atrial appendage occluder in Figure 2;
Figure 12 is a schematic diagram of a test method of the radial deformability of the sealing plate of the left atrial appendage occluder in Figure 2;
Figure 13 is a schematic diagram of another specific test method of the radial deformability of the sealing plate/fixing frame of the left atrial appendage occluder in Figure 2;
Figure 14 is a schematic diagram of a first test method of the axial deformability of the fixing frame of the left atrial appendage occluder in Figure 2;
Figure 15 is a schematic diagram of a first test method of the axial deformability of the sealing plate of the left atrial appendage occluder in Figure 2;
Figure 16 is a schematic diagram of a second test method of the axial deformability of the fixing frame of the left atrial appendage occluder in Figure 2;
Figure 17 is a schematic diagram of a second test method of the axial deformability of the sealing plate of the left atrial appendage occluder in Figure 2.

### Detailed Description of Embodiments

For the purpose of making the description clearer, the present disclosure defines terms "distal end" and "proximal end". The above-mentioned terms are common used in the field of interventional medical devices. To be more specific, the "distal end" represents an end far away from an operator in a surgical process, and the "proximal end" represents an end close to the operator in the surgical process.

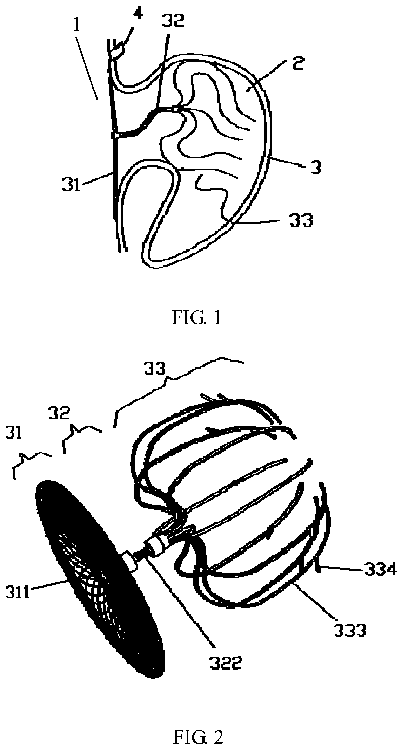

Figure 1 illustrates a schematic diagram of a left atrial appendage occluder implanted into a left atrial appendage according to an embodiment of the present disclosure. The left atrial appendage 2 is located inside a left atrial appendage 1 and between a mitral valve (not shown in figures) and a left superior pulmonary vein 4. With reference to Figure 2 at the same time, the left atrial appendage occluder includes a sealing plate 31, a fixing frame 33 located on one side of the distal end of the sealing plate 31, and a connection member 32 for connecting the sealing plate 31 with the fixing frame 33.

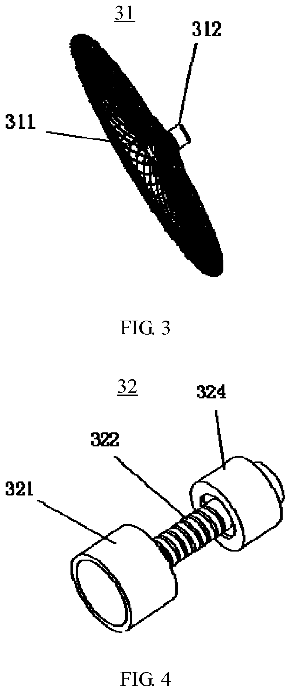

With reference to Figure 3, the sealing plate 31 is a filament woven structure, for example, it may be of a double-layer filament woven structure. The sealing plate 31 may be formed by weaving metal wires (a nickel-titanium material is preferred), and then by a heat treatment. The number of the metal wires is 16 to 144 metal wires, and preferably, for example, 36 to 72 wires, and the diameter of each metal wire is 0.01 mm to 0.8 mm. The sealing plate 31 is substantially a disk shape, and its diameter is greater than the maximum diameter of an opening portion of the left atrial appendage 2. The sealing plate 31 may adapt to left atrial appendages 2 with various opening shapes as long as the specification of the sealing plate 31 selected is large enough to cover the opening portion of each left atrial appendage 2. The sealing plate 31 is arranged at the opening portion of the left atrial appendage 2 and seals the opening portion, which can prevent a flow channel built between the left atrial appendage 2 and the left atrium 1 and can prevent blood from entering the left atrial appendage 1.

Proximal ends of the metal wires of the sealing plate 31 are gathered and fixed by a proximal fixing member 311, and distal ends of the metal wires of the sealing plate 31 are gathered and fixed by a distal fixing member 312. The proximal fixing member 311 and the sealing plate 31 can be fixed in a conventional way such as welding, as well as the distal fixing member 312 and the metal wires of the sealing plate 31. The distal fixing member 312 is connected with the connection member 32. Further, the proximal fixing member 311 has threads, and the threads may be connected with a deliverer or delivery device to deliver the left atrial appendage occluder.

Further, the interior of the sealing plate 31 is provided with a sealing film (not shown in figures); and the size of the sealing film is basically the same as that of the sealing plate 31. To be more specific, the diameter of the sealing film is equal to that of the sealing plate 31. The sealing film is made of a macromolecular material, preferably PTFE (polytetrafluoroethylene) or PET (polyethylene terephthalate). The sealing film may be fixed with the metal wires, which constitute the sealing plate 31, by sewing, or gluing.

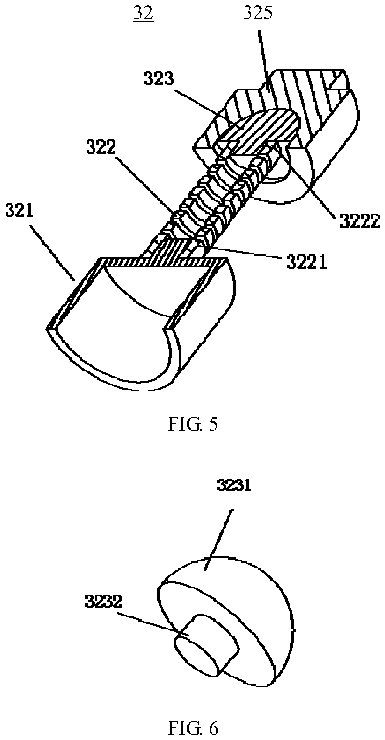

With reference to Figure 4 and Figure 5, the connection member 32 includes a proximal connecting end 321, a distal connecting end 324, and a connecting rod 322 connected between the proximal connecting end 321 and the distal connecting end 324.

The proximal connecting end 321 is connected with the sealing plate 31. To be more specific, the proximal connecting end 321 is connected with the distal fixing member 312 of the sealing plate 31. The connection may be realized by welding, adhering, or of interference fit or other ways.

The distal connecting end 324 is connected with the fixing frame 33. The connection may be realized by welding, adhering, or an interference fit, or other ways.

The distal connecting end 324 includes a ball head structure 323 and a ball socket structure 325 which are matched with each other. A hinge mechanism, formed by the ball head structure 323 and the ball socket structure 325, may adjust an angle within 360 degrees. The ball head structure 323 is connected with the connecting rod 322, and the ball socket structure 325 is connected with the fixing frame 33, so that an angle between the fixing frame 33 and the connection member 32 also may be flexibly adjusted, and the left atrial appendage occluder may adapt to left atrial appendages 2 with different shapes in a wider range.

With reference to Figure 5, Figure 6 and Figure 7 at the same time, the proximal connecting end 321 is connected with the proximal end 3221 of the connecting rod 322 by welding, adhering, or by interference fit, or other ways. The ball head structure 323 includes a convex spherical surface 3231 facing to the ball socket structure 325, and a convex pillar 3232 facing to the connecting rod 322. The ball socket structure 325 includes a convex end 3251 facing to the fixing frame 33 and a concave spherical surface 3252 facing to the ball head structure 323. The distal end 3222 of the connecting rod 322 is connected with the convex pillar 3232 of the ball head structure 323 by welding, adhering, or by interference fit, or other ways. The convex end 3251 of the ball socket structure 325 is connected with the fixing frame 33. The convex spherical surface 3231 is contained in the concave spherical surface 3252 to form a movable connection, so that the ball socket structure 325 and the ball head structure 323 may rotate at any angle.

The connection member 32 may be made of a metal with better biocompatibility, such as stainless steel or a nickel-titanium material. The connecting rod 322 may be a rod structure with a diameter of 0.1 mm to 5 mm, and also may be a spring structure. The connecting rod 322 and the fixing frame 33 have characteristics of hinged active connection, and the connecting rod 322 is also elastic. Therefore, in addition to the feature that a connecting angle between the connection member 32 and the fixing frame 33 may be changed, the length of the connection member 32 also may be adjusted, so that the left atrial appendage occluder provided may adapt to more left atrial appendages in different shapes, and movements of the left atrial appendages, after the left atrial appendage occluder had been implanted into the left atrial appendages.

As shown in Figure 1, after the left atrial appendage occluder is implanted into a left atrial appendage 2 with a relatively large bending angle, the fixing frame 33 needs to find a proper fixing position in the left atrial appendage 2, but there is a relatively large included angle between this fixing position and the opening portion of the left atrial appendage 2. That is, after the left atrial appendage occluder is implanted into the left atrial appendage 2, a relatively large included angle and long distance are formed between the fixing frame 33 and the sealing plate 31. Further, by adjusting the length and the angle of the connection member 32, it is possible to adjust the included angle and the relative distance which are formed between the fixing frame 33 and the sealing plate 31, so that the fixing frame 33 of the left atrial appendage occluder can be stably fixed at a proper position in the left atrial appendage 2, and the sealing plate 31 may be optimally fitted to the opening portion of the left atrial appendage 2 to achieve a sealing effect.

The fixing frame 33 includes a frame structure. The frame structure includes a proximal side surface, and a supporting circumferential surface which is connected with the proximal side surface and extends from the proximal side surface to the distal end; and the connection member is connected with the proximal side surface. In a naturally unfolded state, the proximal side surface may be basically parallel to the sealing plate, and the supporting circumferential surface may be similar to a columnar surface, which includes a proximal opening and a distal opening; the proximal opening is connected with the proximal side surface; and the distal opening is open and suspended.

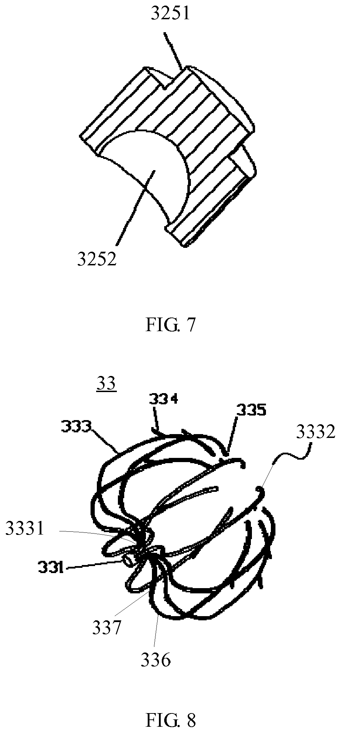

With reference to Figure 8, in one embodiment, the fixing frame 33 includes a fixing connector 331 connected with the connection member 32, and multiple supporting rods 333. The fixing connector 331 is connected with the connection member 32. To be more specific, the distal connecting end 324 of the connection member 32 is connected with the fixing connector 331, for example, the connection may be realized by welding, adhering, or by interference fit, or other ways. The first ends 3331 of the multiple supporting rods 333 are gathered and fixed by the fixing connector 331, and the first end 3331 is the proximal end of the supporting rod 333. The second ends 3332 radially extend out from the fixing connector 331 along a radial direction to form the proximal side surface, and then bend and axially extend, towards the distal end, to form the supporting circumferential surface; the second ends 3332 of the multiple supporting rods 333 extend towards a direction away from the connection member 32 and are separated from one another; and the second end 3332 is the distal end of the supporting rod 333.

By optimizing the structure of the fixing frame of the left atrial appendage occluder, the proximal ends of the supporting rods of the fixing frame are gathered, and are fixed by a fixing connector to realize a closed state, and the distal ends of the supporting rods are separated from one another to realize an open state, so that a sufficient supporting force of the fixing frame may be guaranteed, and each supporting rod has certain relative independence, and it may maintain relatively good fitness with a cavity wall of a left atrial appendage to enhance a stable performance of the fixing frame in the left atrial appendage. With the above capabilities, the fixing frame may be also stably fixed in the left atrial appendage without anchor bars. Because of the smooth surface of the fixing frame, the fixing frame will not pierce the cavity wall of the left atrial appendage even if the fixing frame is arranged at a relatively deep position (where the cavity wall here is relatively thin) of the left atrial appendage.

To be more specific, with reference to Figure 8, each supporting rod 333 includes a proximal arc section 337, a distal arc section 335, and a middle arc section 336 connected between the proximal arc section 337 and the distal arc section 335. The proximal ends of the proximal arc sections 337 are connected with the fixing connector 331. The distal ends of the distal arc sections 335 are the second ends 3332 of the supporting rods 333. In the naturally unfolded state of the fixing frame 33, the multiple supporting rods 333 form an approximately spherical space in an encircling manner. It can deem that the contour of this approximately spherical space is approximately a sphere, but not a completely regular sphere. In this embodiment, in order to enable the supporting rods 333 to have higher supporting strength, the proximal arc sections 337 are designed to protrude away from the connection member 32, that is, the proximal arc sections 337 protrude towards the distal end of the fixing frame 33; the middle arc sections 336 protrude towards the connection member 32, that is, the middle arc sections 336 protrude away from the distal end of the fixing frame 33; the proximal arc section 337 and the middle arc section 336 form an approximately S-shaped curve; and the distal arc sections 335 protrude away from the center of the approximately spherical space, wherein the distal arc sections 335 are main components for forming the spherical shape in this embodiment, and when contacted with the cavity wall 3 of the left atrial appendage 2, the distal arc sections 335 are also main contact components. The distal arc sections 335 of the fixing frame 33 protrude away from the center of the approximately spherical space, so that the distal ends of the distal arc sections 335 are restrained to avoid sharp edges, so as to prevent the left atrial appendage occluder damaging the left atrial appendage 2.

There are at least two supporting rods 333, and preferably six supporting rods, to ensure that the fixing frame 33 and the cavity wall 3 of the left atrial appendage 2 have enough contact pivots and enough contact area to guarantee the stable fixing effect.

In some embodiments, anchor bars 334 are arranged on the distal arc sections 335. The anchor bars 334 may face to the sealing plate. The anchor bars 334 are used for puncturing the cavity wall 3 of the left atrial appendage 2 to assist the fixing frame 33 in being fixed. As the fixing frame 33 may be firmly connected with the cavity wall 3 of the left atrial appendage 2 by its structure, of which one end is closed and the other end is open, it can be understood that the anchor bars 334 on the fixing frame 33 may be omitted in some embodiments, as desired, to avoid piercing the cavity wall 3 of the left atrial appendage 2.

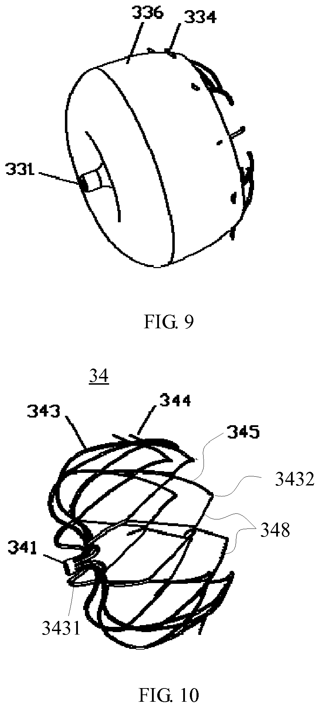

With reference to Figure 9, in some other embodiments, the fixing frame 33 also may include a film 336. The film 336 at least covers a part, which has the anchor bars 334, of the fixing frame 33. The film 336 also may cover the whole outer surface of the fixing frame 33. When the specification is improper, the film 336 arranged on the outer surface of the fixing frame 33 may prevent such a phenomenon where blood flows into the pericardium due to the fact that the anchor bars 334 pierce the cavity wall 3 of the left atrial appendage 2. Meanwhile, the film 336 is also a second seal between the left atrial appendage 2 and the left atrium 1 to prevent a circulation between the left atrial appendage 2 and the left atrium 1. The film 336 is made of a polymer material which may be the PTFE or the PET. The film 336 may be attached to the fixing frame 33 by swing or gluing.

The fixing frame 33 may be formed by heat treatment after cutting a metal tube. To be more specific, the fixing frame 33 may be formed by cutting a metal (preferably a nickel-titanium material) tube with a diameter of 0.3 mm to 5 mm and a length of 10 mm to 50 mm into a certain pattern, and then shaped by heat treatment. In other words, the fixing connector 331 and the supporting rods 333 may be integrated.

In some other embodiments, the fixing frame 33 also may be formed by connecting multiple metal wires after these metal wires had been heat treated. For example, the fixing frame 33 may be formed by connecting multiple metal wires with diameters of 0.05 mm to 0.8 mm or flat metal wires with sectional areas of (0.03 mm to 0.5 mm) × (0.5 mm to 0.03 mm) after these metal wires had been heat treated, and the metal is preferably nickel-titanium. The proximal ends of the metal wires may be fixed by welding to form the fixing connector 331.

As shown in Figure 10, in some other embodiments, the shape and structure of a fixing frame 34 are similar to those of the fixing frame 33 as shown in Figure 8. To be more specific, this fixing frame 34 also has a fixing connector 341 and multiple supporting rods 343. The first ends 3431 of the supporting rods 343 are gathered and fixed by the fixing connector 341, and the second ends 3432 of the supporting rods 343 extend towards a direction away from the connection member 32 and are separated from one another. Anchor bars 344 are arranged on the supporting rods 343. The difference is that the fixing frame 34 also includes branches 348 connected with two adjacent supporting rods 343. The branches 348 may restrict the distal arc sections 345 of the supporting rods 343 to a certain extent, and can form grids, so that the distal arc sections 345 are unlikely to deform and expand outward when compressed, and the risk of piercing the cavity wall 3 of the left atrial appendage 2 is reduced. In addition, by the arrangement of the branches 348, the supporting force of the fixing frame 34 may be also increased to a certain extent, to make the fixing frame 34 firmly fix in the left atrial appendage 2.

Preferably, connection nodes between the branches 348 and the supporting rods 343 are located in the middle portions and/or the distal ends of the distal arc sections 345. In one specific embodiment, two branches 348 are connected between two adjacent supporting rods 343; and the two branches 348 are inclined relative to the supporting rods 343, and extending directions of the two branches are roughly parallel, wherein one branch 348 is connected between the distal arc sections 345 of two adjacent supporting rods 343, and one end of the other branch 348 is connected to the middle portion of the distal arc section 345 of one supporting rod 343, and the other end of the other branch 348 is connected to the distal end of the distal arc section 345, namely the second end 3432, of the other supporting rod 343.

According to the invention the deformability of the sealing plate 31 is greater than that of the fixing frame 33. The deformability of a certain component or structure is the magnitude of a deformation amount of this component or structure under the action of an external force. In this present disclosure, the deformability described herein may be expressed by a radial length (for example the diameter) variation of the component or structure under the action of a radial force.

Further, the radial deformability of the sealing plate 31 of the left atrial appendage occluder is greater than that of the fixing frame 33, and/or the axial deformability of the sealing plate 31 is greater than that of the fixing frame 33. To be more specific, under the action of the same radial force, the radial length variation of the sealing plate 31 is greater than that of the fixing frame 33; or under the action of the same radial force, a radial length change rate of the sealing plate 31 is greater than that of the fixing frame 33; or under the action of the same axial force, a displacement of the sealing plate 31 along an axial force direction is greater than that of the fixing frame.

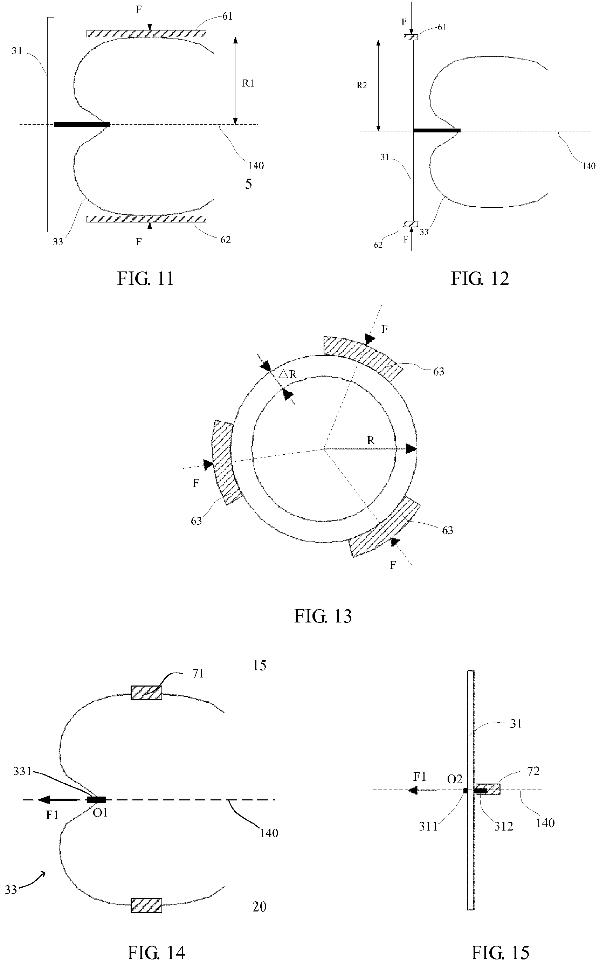

Radial length changes of the fixing frame and the sealing plate under the action of the same radial force may be respectively tested by adopting a flat plate method. For example, with reference to Figure 11 and Figure 12, the left atrial appendage occluder may be tested by the flat plate method.

With reference to Figure 11, first, on the premise that the sealing plate 31 maintains a freely unfolded state, radial acting forces F are applied to the fixing frame 33 by two parallel flat plates 61 and 62. To be more specific, the parallel flat plates 61 and 62 are respectively placed on two opposite sides of a diameter of the fixing frame 33, and two radial acting forces F with the same size and opposite directions are respectively applied to the flat plates 61 and 62 along the diameter. The diameter of the fixing frame 33 penetrates through and is perpendicular to a central axial line 140. The two parallel flat plates 61 and 62 maintain a mutually parallel state in the whole test process, namely the flat plates are parallel to the central axial line 140 all the time in the test process. And any one of the flat plates at least covers a contour with maximum radius of the fixing frame 33, and preferably the flat plate covers the whole fixing frame 33 in a direction parallel to the central axial line 140. In the naturally unfolded state, the radial lengths of portions, where the flat plates are loaded, of the fixing frame 33 are R1, the radial length variation of the fixing frame 33, under the action of the radial forces F, is a radial length difference obtained before and after radial compression, and may be expressed by ΔR1, so that the radial length change rate is ΔR1/R1. In order to guarantee that the flat plate itself is not deformed in a radial force applying process, and the radial forces can be evenly applied to all portions of the flat plates, a thickness of the flat plate is at least 5 mm.

With reference to Figure 12, the sealing plate 31 is tested by the flat plate method as above, namely the same radial acting forces F which refer to the same size, direction and acting time, are adopted. On the premise that the sealing plate 31 is naturally unfolded, the radial length variation ΔR2 or the radial length change rate ΔR2/R2 of the sealing plate 31 is tested; and at the moment, a contour with maximum radius of the sealing plate 31 is located at the edges of the double plates. Based on the above test condition, under the action of the same radial force, the radial length variation ΔR2 of the sealing plate 31 of the left atrial appendage occluder according to the embodiment of the present disclosure is greater than the radial length variation ΔR1 of the fixing frame 33; or the radial length change rate ΔR2/R2 of the sealing plate 31 of the left atrial appendage occluder according to the embodiment of the present disclosure is greater than the radial length change rate ΔR1/R1 of the fixing frame 33.

After the left atrial appendage occluder is implanted into a human body, a situation where the implantation location may be improperly selected, may occur. For example, if the fixing frame extends too deep into the cavity of the left atrial appendage, an axial length of the occluder in the naturally unfolded state would be shorter than a relative distance between the fixing frame and the sealing plate after implantation, which will lead to a mutual traction between the fixing frame and the sealing plate. Or, the implanted occluder would move together with the heart, the implanted occluder and the heart have different amplitudes or directions of motion, which may lead to the mutual traction between the fixing frame and the sealing plate. Generally, the fixing frame and the sealing plate pull each other through the connection member.

When the fixing frame is pulled by the sealing plate, as the fixing frame is fixed in the cavity of the left atrial appendage through a radial supporting force surrounding a circumferential region of the central axial line 140, the fixing frame is attached to the circumferential region of the cavity of the left atrial appendage to resist this pulling acting force. Therefore, the fixing frame will be radially deformed under the axial acting force, and the fixing frame will separate from the cavity wall of the left atrial appendage if the acting force is large enough, and then the left atrial appendage occluder would fall off, which causes an implantation failure. When the sealing plate is pulled by the fixing frame, the sealing plate has a disk surface structure and is connected with the connection member through the disk surface, so that the axial pulling on the sealing plate would also lead to a radial deformation of the sealing plate.

Therefore, when the fixing frame and the sealing plate pull each other, the one who is easily deformed in the radial direction will be pulled by the other one. For example, under the same radial acting force, as the radial length variation of the fixing frame according to the embodiment of the present disclosure is less than that of the sealing plate, or the radial length change rate of the fixing frame according to the embodiment of the present disclosure is less than that of the sealing plate, in the mutual traction, the fixing frame would dominate the traction and pull the sealing plate to make the sealing plate deform towards the fixing frame direction (or towards the distal end). Such deformation enables the sealing plate to be more close to a left atrial wall at an opening of the left atrial appendage than that of the sealing plate in the naturally unfolded state, which enhances a sealing effect between the sealing plate and the opening of the left atrial appendage and avoids an interval between the sealing plate and the left atrial wall, so that it can prevent apoplexy or systematic embolism caused by blood flowing into the cavity of the left atrial appendage and a thrombus flowing into a left atrium through the interval. In addition, the fixing frame dominates the traction so that the fixing frame will not easily separate from the cavity wall of the left atrial appendage under the pull of the sealing plate, and the occluder will be better fixed in the left atrial appendage to avoid falling off from the left atrial appendage.

The above flat plate method is only an example method, and not a limitation of the present disclosure. An person of ordinary skill in the art can adopt any other proper method, equivalent to the flat plate method, for testing. For example, a test method that the radial acting force is evenly applied to a tested component in a circumferential direction. To be more specific, with reference to Figure 13, three arc-shaped plates 63 may be uniformly disposed, in the same circumferential direction, on the contour with maximum radius of the tested component (the fixing frame or the sealing plate). During the test, radial acting forces F are simultaneously applied to the above arc-shaped plates 63 along a radial direction to test a variation or a change rate of a radial length R of the tested component. Similarly, in order to apply the radial force evenly, a thickness of the arc-shaped plates is at least 5 mm. Furthermore, the left atrial appendage occluder may be tested by a radial supporting force tester RX550-100 of the Machine Solution Inc (MSI) Company.

In addition, when one portion of the tested component (the fixing frame or the sealing plate) is restricted, the axial deformability of the tested component is expressed by testing an axial displacement (along the direction of the central axial line 140) of the tested component under an action of the same axial force. In the test method as above, the above restriction is an equidimensional restriction, that is to say, in the restriction process, the tested component does not elastically deform or deforms only a little, which may be ignored; and, in addition, the axial acting force is applied to a position, where no elastic deformation occurs, of the tested component. For example, the same axial acting forces are applied to end portions, which are connected with the connection member, of the tested component, and the axial displacement of the tested component is used to express its own deformability, and the axial displacement of the component here refers to a position of the tested component where the axial acting force is applied, and the left atrial appendage meets the condition that the axial displacement of the fixing frame is less than that of the sealing plate. During the testing, the fixing frame and the sealing plate are independently tested, for example, only a single fixing frame or a single sealing plate is tested at each time.

With reference to Figure 14, during a test of the fixing frame 33, a clamping portion, where the fixing frame 33 is clamped by an annular clamping member 71 along a circumferential direction, is the contour with maximum radius of the fixing frame 33. The annular clamping member 71 surrounds and is perpendicular to the central axial line 140. In a clamping process, the radial size of the clamping portion of the fixing frame 33 is basically equal to that of a fixing frame 33 in the naturally unfolded state, so that the elastic deformation may be ignored. An axial acting force F1, along the central axial 140 and towards the direction of the sealing plate 31, is applied to the fixing connector 331, where the fixing frame 33 is connected with the connection member, and the fixing connector 331 does not deform under the axial acting force F1. Measuring an axial displacement ΔO1 of a projection O1 of the fixing connector 331 on the central axial line 140 under the F1. The axial displacement ΔO1 is used to express the deformation (or the deformability) of the fixing frame 33, and the state of the clamping member 71 itself maintains unchanged during the action of the axial acting force F1.

After the left atrial appendage occluder is implanted into the human body, and under a condition that one portion, such as the maximum contour, of the fixing frame is clamped, it can be seen that the tested axial displacement, under the action of the axial acting force, represents the axial deformability of the fixing frame, which is under the pull of the sealing plate and the restricting action of the cavity of the left atrial appendage. Under the same axial acting force, the larger the axial displacement ΔO1 is, the easier it is for the fixing frame to be pulled and deformed.

With reference to Figure 15, during a test of the sealing plate 31, the distal fixing member 312 of the sealing plate 31 is clamped by the clamping member 72; and the axial acting force F1, applied to the proximal fixing member 311 of the sealing plate 31 along the central axial line 140 and towards a direction away from the fixing frame 33, is totally equal to the test of the fixing frame 33. Measuring another axial displacement ΔO2 of a projection O2 of the proximal fixing member 311 on the central axial line 140 under the F1, and the axial displacement ΔO2 is used to express the deformation (or the deformability) of the sealing plate 31.

After the left atrial appendage occluder is implanted into the human body, and under a condition that one portion, such as the distal fixing member 312, of the sealing plate 31 is clamped, it can be seen that the tested axial displacement under the action of the axial acting force F1 represents the axial deformability of the sealing plate 31, which is under the pull of the fixing frame 33 and the restricting action of a tissue wall of the opening portion of the left atrial appendage. Under the same axial acting force, the larger the axial displacement ΔO2 is, the easier the sealing plate 31 will be pulled and deformed.

It is tested that, under the action of the same axial force, the axial displacement ΔO1 of the fixing frame is less than the axial displacement ΔO2 of the sealing plate. It can be understood that when the fixing frame and the sealing plate pull each other, the one who has a larger axial displacement will be pulled by the other one. For example, under the same axial acting force, as the axial displacement of the fixing frame according to the embodiment of the present disclosure is less than that of the sealing plate, the fixing frame would dominate the traction and pull the sealing plate during the traction to make the sealing plate deform towards a direction of the fixing frame (or towards the distal end). Such deformation enables the sealing plate to be more close to the left atrial wall at the opening of the left atrial appendage than that of the sealing plate in the naturally unfolded state, which enhances a sealing effect between the sealing plate and the opening of the left atrial appendage and avoids an interval between the sealing plate and the left atrial wall, so that it can prevent blood from flowing into the cavity of the left atrial appendage and a thrombus from flowing into a left atrium through the interval. In addition, the fixing frame dominates the traction so that the fixing frame will not easily separate from the cavity wall of the left atrial appendage under the pull of the sealing plate, and the occluder will be better fixed in the left atrial appendage to avoid falling off from the left atrial appendage.

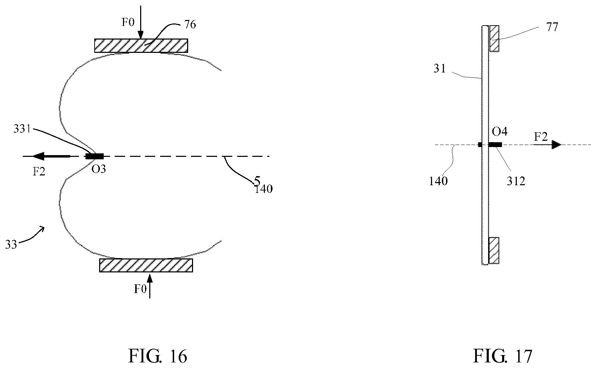

A second axial deformability test method may be further adopted. With reference to Figure 16, during a test of the fixing frame 33, a clamping portion, where the fixing frame 33 is clamped by an annular clamping member 76 along a circumferential direction, is a contour with maximum radius of the fixing frame 33. The annular clamping member surrounds and is perpendicular to the central axial line 140. In a clamping process, the radial size of the clamping portion of the fixing frame 33 is smaller than that of a fixing frame 33 in the naturally unfolded state. The clamping portion of the fixing frame 33 is compressed in the radial direction, for example, so that the maximum radial length after compression is 80 percent of the maximum radial length before it is compressed. Of course, other compression ratios may be adopted, as desired, which are not listed one by one here. For example, a radial force F0 may be applied to the annular clamping member 76 to compress the fixing frame 33 in the radial direction. An axial acting force F2 is applied to the fixing connector 331, where the fixing frame 33 is connected with the connection member, and the fixing connector 331 does not deform under the axial acting force F2 which is along the central axial line 140 and towards the direction of the sealing plate 31. Measurement of an axial displacement ΔO3 of a projection O3 of the fixing connector 331 on the central axial line 140 along under the F2 may be performed. The axial displacement ΔO3 is used to express the deformation (or the deformability) of the fixing frame 33.

After the left atrial appendage occluder is implanted into a human body, and under a condition that one portion, such as the maximum contour, of the fixing frame 33 is clamped, it can be seen that the tested axial displacement under the action of the axial acting force represents the deformability of the fixing frame 33, which is under the pull of the sealing plate 31 and the restricting action of the cavity of the left atrial appendage. Under the same axial acting force, the larger the axial displacement ΔO3 is, the easier it is for the fixing frame 33 to be pulled and deformed.

With reference to Figure 17, the sealing plate 31 includes a distal fixing member 312; and the connection member is connected with the distal fixing member 312. During an independent test of the sealing plate 31, an annular fixing member 77 abuts a disk surface, which is towards the fixing frame 33 and located at the maximum edge of the sealing plate 31. Meanwhile, the axial acting force F2, along the central axial line 140 and towards a direction of the fixing frame 33, is applied to the distal fixing member 312. Under the axial acting force F2, a position where the disk surface is abutted maintains unchanged along the direction of the central axial line 140 due to the annular fixing member 77, thereby testing a projection displacement ΔO4 of the distal fixing member 312 on the central axial line 140.

It can be seen from the above that after the left atrial appendage occluder is implanted into the human body, a portion of the sealing plate is blocked by the cavity wall of the left atrium at the opening portion of the left atrial appendage, wherein the portion of the sealing plate faces to the fixing frame and is at least the edge of the sealing plate with maximum radius. Therefore, during the above test of the sealing plate, and under a condition that the position, abutted by the annular fixing member 77, of the disk surface maintains unchanged along the direction of the central axial line 140, and an axial displacement, tested under the axial acting force, of the sealing plate represents the deformability of the sealing plate pulled by the fixing frame at the opening of the left atrial appendage after the occluder is implanted into the human body. Under the same axial acting force, the larger the axial displacement ΔO4 is, the easier the sealing plate will be pulled and deformed.

It is tested that, under the action of the same axial force (F2), the axial displacement ΔO3 of the fixing frame is less than the axial displacement ΔO4 of the sealing plate. It can be understood that when the fixing frame and the sealing plate pull each other, the one who has a larger axial displacement will be pulled by the other one. For example, under the same axial acting force, as the axial displacement of the fixing frame according to the embodiment of the present disclosure is less than that of the sealing plate, the fixing frame would dominate the traction and pull the sealing plate during the traction to make the sealing plate deform towards a direction of the fixing frame (or towards the distal end). Such deformation enables the sealing plate to be closer to the left atrial wall at the opening of the left atrial appendage than that of the sealing plate in the naturally unfolded state, which enhances a sealing effect between the sealing plate and the opening of the left atrial appendage and avoids an interval between the sealing plate and the left atrial wall, so that it can prevent blood from flowing into the cavity of the left atrial appendage and a thrombus from flowing into a left atrium through the interval. In addition, the fixing frame dominates the traction so that the fixing frame will not easily separate from the cavity wall of the left atrial appendage under the pull of the sealing plate, and the occluder will be better fixed in the left atrial appendage to avoid falling off from the left atrial appendage.

Above all, the left atrial appendage occluder provided by the present disclosure has the following beneficial effects:
(1) the fixing frame is of the structure, which includes at least two supporting rods and has one closed end and one open end, and the fixing frame is flexible and has a smooth outer surface. When placed at a proper position in the left atrial appendage, the fixing frame may be compressed, and its supporting rods may provide a relatively high supporting force to the left atrial appendage, to guarantee stable fixing, and avoid injury to the cavity wall of the left atrial appendage. The anchor bars further arranged on the fixing frame may puncture the wall of the left atrial appendage conveniently, which is helpful to fix the left atrial appendage occluder. The film attached to the fixing frame may prevent a phenomenon where blood flows into the pericardium due to the fact that the anchor bars pierce the wall of the left atrial appendage. This film is a second seal between the left atrial appendage and the left atrium as well to prevent a circulation between the left atrial appendage and the left atrium.
(2) The sealing plate is a disk shape woven by multiple metal wires and has good elasticity or elastic properties. The sealing plate is arranged at the opening portion of the left atrial appendage and may be well fitted to the opening portion of the left atrial appendage to achieve the best sealing effect. The thread is formed at the proximal end of the sealing plate to realize connection with a deliverer or delivery device.
(3) The connection member has elasticity and relatively high bending resistance, so that it is able to adjust the length and the angle between the sealing plate and the fixing frame; and in a situation where the sealing plate and the fixing frame are not coaxial, and it is required by the anatomical structure of the left atrial appendage, the length and the angle between the sealing plate and the fixing frame may be adjusted, so that the stable fixing effect is guaranteed, and the optimal sealing effect may be achieved.

## Claims

1. A left atrial appendage occluder, comprising:
a sealing plate (31), a fixing frame (33) located on one side of the distal end of the sealing plate (31), and a connection member (32) for connecting the sealing plate (31) with the fixing frame (33), wherein the fixing frame (33) comprises a frame structure;
the frame structure comprises a proximal side surface and a supporting circumferential surface which is connected with the proximal side surface and extends from the proximal side surface to the distal end, and the connection member (32) is connected with the proximal side surface,
**characterized in that** the radial deformability of the sealing plate (31) is greater than that of the fixing frame (33) and/or the axial deformability of the sealing plate (31) is greater than that of the fixing frame (33).

2. The left atrial appendage occluder according to claim 1, wherein, under an action of a same radial force, a radial length variation of the sealing plate (31) is greater than that of the fixing frame (33); or
under an action of a same radial force, a radial length change rate of the sealing plate (31) is greater than that of the fixing frame (33); or
under the action of the same axial force, a displacement of the sealing plate (31) along an axial force direction is greater than that of the fixing frame (33) along the axial force direction.

3. The left atrial appendage occluder according to claim 1, wherein, in a naturally unfolded state of the left atrial appendage occluder, the proximal side surface is substantially parallel to the sealing plate (31);
the supporting circumferential surface is similar to a columnar surface;
a proximal opening of the supporting circumferential surface is connected with the proximal side surface; and
a distal opening of the supporting circumferential surface is opened in an impending manner.

4. The left atrial appendage occluder according to claim 3, wherein the frame structure comprises a proximal end and a plurality of elastic supporting rods (334);
ends of elastic supporting rods (333) are connected with the proximal end in a gathering manner, and the other ends of the elastic supporting rods extend out from the proximal end in a radial manner along a radial direction to form the proximal side surface, and then bend, and axially extend towards the distal end to form the supporting circumferential surface; or
and at least one of the proximal side surface and the supporting circumferential surface comprises a plurality of grids formed by the elastic supporting rods (334) mutually connected in an encircling manner.

5. The left atrial appendage occluder according to claim 1, wherein the left atrial appendage occluder further comprises at least one anchor bar (334) arranged on the frame structure and facing to the sealing plate (31).

6. The left atrial appendage occluder according to claim 1, wherein the fixing frame (33) further comprises a film (356) which is arranged on the frame structure and at least covers the supporting circumferential surface.

7. The left atrial appendage occluder according to claim 6, wherein the fixing frame (33) further comprises at least one anchor bar (334) which is arranged on the supporting circumferential surface and faces to the sealing plate (31), and the at least one anchor bar penetrates through the film (356).

8. The left atrial appendage occluder according to claim 1, wherein the connection member (32) is a flexible connection member or an elastic connection member.

9. The left atrial appendage occluder according to claim 8, wherein the connection member (32) comprises a proximal connecting end (321), a distal connecting end (324) and a connecting body (322) connected between the proximal connecting end and the distal connecting end;
the proximal connecting end (321) is connected with the sealing plate (31);
the distal connecting end (324) is connected with the fixing frame (33);
the distal connecting end includes a ball socket (325); and
the distal end of the connecting body includes a ball head 323) matched with the ball socket.

10. The left atrial appendage occluder according to claim 9, wherein the connecting body (322) is an elastic or flexible rod, or a spring structure, or a woven structure formed by a plurality of elastic filaments.

11. The left atrial appendage occluder according to claim 1, wherein the sealing plate (31) is of a double-layer filament woven structure.

12. The left atrial appendage occluder according to claim 4, wherein the supporting rods (334) form an approximately spherical space, each of the supporting rods (334) comprises a proximal arc section(337), a distal arc section (335), and a middle arc section (336) connected between the proximal arc section and the distal arc section, and the proximal arc section is connected with a fixing connector (331);
the proximal arc section (337) protrudes towards the distal end of the fixing frame (33);
the middle arc section (336) protrudes away from the distal end of the fixing frame (33);
the proximal arc section and the middle arc section form an approximately S-shaped curve; and
the distal arc section (335) protrudes away from a center of the approximately spherical space.

13. The left atrial appendage occluder according to claim 12, wherein the fixing frame (33) further comprises a branch (348) connected with two adjacent supporting rods (334), and connection nodes between the branches and the supporting rods (334) are located in the middle portions and/or the distal ends of the distal arc sections (335).

14. The left atrial appendage occluder according to claim 13, wherein two branches (348) are connected between two adjacent supporting rods (334); and
the two branches (348) are inclined relative to the supporting rods (334), and extending directions of the two branches are roughly parallel, wherein one branch is connected between the distal arc sections (335) of two adjacent supporting rods (334), and one end of the other branch is connected to the middle portion of the distal arc section of one supporting rod, and the other end of the other branch is connected to the distal end of the distal arc section.

## Patentansprüche

1. Verschluss für das linke Herzohr, mit:
einer Verschlussplatte (31), einem Fixierrahmen (33), der sich auf einer Seite des distalen Endes der Verschlussplatte (31) befindet, und einem Verbindungselement (32) zum Verbinden der Verschlussplatte (31) mit dem Fixierrahmen (33), wobei der Fixierrahmen (33) eine Rahmenstruktur umfasst,
wobei die Rahmenstruktur eine proximale Seitenfläche und eine stützende Umfangsfläche umfasst, die mit der proximalen Seitenfläche verbunden ist und sich von der proximalen Seitenfläche zum distalen Ende erstreckt, und das Verbindungselement (32) mit der proximalen Seitenfläche verbunden ist,
**dadurch gekennzeichnet, dass** die radiale Verformbarkeit der Verschlussplatte (31) größer als die des Fixierrahmens (33) ist und/oder die axiale Verformbarkeit der Verschlussplatte (31) größer als die des Fixierrahmens (33) ist.

2. Verschluss für das linke Herzohr nach Anspruch 1, wobei unter Einwirkung einer gleichen Radialkraft eine Veränderung der radialen Länge der Verschlussplatte (31) größer als die des Fixierrahmens (33) ist, oder
unter Einwirkung einer gleichen Radialkraft eine Änderungsrate der radialen Länge der Verschlussplatte (31) größer als die des Fixierrahmens (33) ist, oder
unter der Einwirkung der gleichen Axialkraft eine Verlagerung der Verschlussplatte (31) längs einer Axialkraftrichtung größer als die des Fixierrahmens (33) längs der Axialkraftrichtung ist.

3. Verschluss für das linke Herzohr nach Anspruch 1, wobei in einem natürlich entfalteten Zustand des Verschlusses für das linke Herzohr die proximale Seitenfläche im Wesentlichen parallel zur Verschlussplatte (31) ist,
die stützende Umfangsfläche einer säulenartigen Fläche ähnlich ist,
eine proximale Öffnung der stützenden Umfangsfläche mit der proximalen Seitenfläche verbunden ist und
eine distale Öffnung der stützenden Umfangsfläche in einer schwebenden Weise geöffnet ist.

4. Verschluss für das linke Herzohr nach Anspruch 3, wobei die Rahmenstruktur ein proximales Ende und mehrere elastische Stützstäbe (334) umfasst,
wobei Enden der elastischen Stützstäbe (333) in zusammenführender Weise mit dem proximalen Ende verbunden sind und die anderen Enden der elastischen Stützstäbe sich zur Bildung der proximalen Seitenfläche von dem proximalen Ende aus radial längs einer Radialrichtung erstrecken und sich dann biegen und sich zur Bildung der stützenden Umfangsfläche axial zum distalen Ende hin erstrecken, oder
und die proximale Seitenfläche und/oder die stützende Umfangsfläche mehrere Gitter umfasst bzw. umfassen, die von den in umschließender Weise miteinander verbundenen elastischen Stützstäben (334) gebildet sind.

5. Verschluss für das linke Herzohr nach Anspruch 1, wobei der Verschluss für das linke Herzohr ferner wenigstens einen Verankerungsstab (334) umfasst, der an der Rahmenstruktur angeordnet und der Verschlussplatte (31) zugewandt ist.

6. Verschluss für das linke Herzohr nach Anspruch 1, wobei der Fixierrahmen (33) ferner eine Folie (356) umfasst, die an der Rahmenstruktur angeordnet ist und zumindest die stützende Umfangsfläche abdeckt.

7. Verschluss für das linke Herzohr nach Anspruch 6, wobei der Fixierrahmen (33) ferner wenigstens einen Verankerungsstab (334) umfasst, der an der stützenden Umfangsfläche angeordnet und der Verschlussplatte (31) zugewandt ist, und der wenigstens eine Verankerungsstab die Folie (356) durchdringt.

8. Verschluss für das linke Herzohr nach Anspruch 1, wobei das Verbindungselement (32) ein flexibles Verbindungselement oder ein elastisches Verbindungselement ist.

9. Verschluss für das linke Herzohr nach Anspruch 8, wobei das Verbindungselement (32) ein proximales Verbindungsende (321), ein distales Verbindungsende (324) und einen Verbindungskörper (322) umfasst, der zwischen dem proximalen Verbindungsende und dem distalen Verbindungsende angeschlossen ist,
das proximale Verbindungsende (321) mit der Verschlussplatte (31) verbunden ist,
das distale Verbindungsende (324) mit dem Fixierrahmen (33) verbunden ist,
das distale Verbindungsende eine Kugelpfanne (325) aufweist und
das distale Ende des Verbindungskörpers einen Kugelkopf (323) aufweist, der mit der Kugelpfanne zusammenpasst.

10. Verschluss für das linke Herzohr nach Anspruch 9, wobei der Verbindungskörper (322) ein elastischer oder flexibler Stab oder eine Federstruktur oder eine aus mehreren elastischen Filamenten gebildete Webstruktur ist.

11. Verschluss für das linke Herzohr nach Anspruch 1, wobei die Verschlussplatte (31) eine Doppellagenfilamentwebstruktur hat.

12. Verschluss für das linke Herzohr nach Anspruch 4, wobei die Stützstäbe (334) einen annähernd kugelförmigen Raum bilden, jeder der Stützstäbe (334) einen proximalen Bogenabschnitt (337), einen distalen Bogenabschnitt (335) und einen mittleren Bogenabschnitt (336) umfasst, der zwischen dem proximalen Bogenabschnitt und dem distalen Bogenabschnitt angeschlossen ist, und der proximale Bogenabschnitt mit einem Fixierverbindungsstück (331) verbunden ist,
der proximale Bogenabschnitt (337) zum distalen Ende des Fixierrahmens (33) hin ragt,
der mittlere Bogenabschnitt (336) vom distalen Ende des Fixierrahmens (33) weg ragt,
der proximale Bogenabschnitt und der mittlere Bogenabschnitt eine annähernd S-förmige Kurve bilden und
der distale Bogenabschnitt (335) von einer Mitte des annähernd kugelförmigen Raums weg ragt.

13. Verschluss für das linke Herzohr nach Anspruch 12, wobei der Fixierrahmen (33) ferner einen mit zwei benachbarten Stützstäben (334) verbundenen Zweig (348) umfasst und Verbindungsknoten zwischen den Zweigen und den Stützstäben (334) in den mittleren Abschnitten und/oder den distalen Enden der distalen Bogenabschnitte (335) liegen.

14. Verschluss für das linke Herzohr nach Anspruch 13, wobei zwei Zweige (348) zwischen zwei benachbarten Stützstäben (334) verbunden sind, und
die beiden Zweige (348) bezüglich der Stützstäbe (334) geneigt sind und Erstreckungsrichtungen der beiden Zweige ungefähr parallel sind, wobei ein Zweig zwischen den distalen Bogenabschnitten (335) zweier benachbarter Stützstäbe (334) verbunden ist und ein Ende des anderen Zweigs mit dem mittleren Abschnitt des distalen Bogenabschnitts eines Stützstabs verbunden ist und das andere Ende des anderen Zweigs mit dem distalen Ende des distalen Bogenabschnitts verbunden ist.

## Revendications

1. Dispositif d'occlusion d'appendice auriculaire gauche, comprenant :
une plaque de fermeture (31), un cadre de fixation (33) agencé d'un côté de l'extrémité distale de la plaque de fermeture (31), et un élément de raccordement (32) pour le raccordement de la plaque de fermeture (31) au cadre de fixation (33), le cadre de fixation (33) comprenant une structure de cadre ;
la structure de cadre comprenant une surface latérale proximale et une surface circonférentielle de support qui est raccordée à la surface latérale proximale et qui s'étend de la surface latérale proximale à l'extrémité distale, et l'élément de raccordement (32) étant raccordé à la surface latérale proximale,
**caractérisé en ce que** la déformabilité radiale de la plaque de fermeture (31) est supérieure à celle du cadre de fixation (33), et/ou la déformabilité axiale de la plaque de fermeture (31) est supérieure à celle du cadre de fixation (33).

2. Dispositif d'occlusion d'appendice auriculaire gauche selon la revendication 1, une variation de la longueur radiale de la plaque de fermeture (31) étant supérieure à celle du cadre de fixation (33) sous l'effet d'une force radiale identique ; ou
un taux de changement de la longueur radiale de la plaque de fermeture (31) étant supérieur à celui du cadre de fixation (33) sous l'effet d'une force radiale identique ; ou
un déplacement de la plaque de fermeture (31) selon un sens de force axiale étant supérieur à celui du cadre de fixation (33) selon le sens de force axiale sous l'effet de la même force axiale.

3. Dispositif d'occlusion d'appendice auriculaire gauche selon la revendication 1, la surface latérale proximale étant sensiblement parallèle à la plaque de fermeture (31) dans un état naturellement déplié du dispositif d'occlusion d'appendice auriculaire gauche ;
la surface circonférentielle de support étant similaire à une surface colonnaire ;
une ouverture proximale de la surface circonférentielle de support étant raccordée à la surface latérale proximale ; et
une ouverture distale de la surface circonférentielle de support étant ouverte de manière imminente.

4. Dispositif d'occlusion d'appendice auriculaire gauche selon la revendication 3, la structure de cadre comprenant une extrémité proximale et une pluralité de tiges de support élastiques (334) ;
des extrémités de tiges de support élastiques (333) étant raccordées à l'extrémité proximale de manière à s'assembler, et les autres extrémités des tiges de support élastiques s'étendant depuis l'extrémité proximale radialement selon un sens radial de manière à former la surface latérale proximale, et étant ensuite pliées, et s'étendant axialement vers l'extrémité distale de manière à former la surface circonférentielle de support ; ou
et la surface latérale proximale et/ou la surface circonférentielle de support comprenant une pluralité de grilles formées par les tiges de support élastiques (334) reliées les unes aux autres de manière encerclante.

5. Dispositif d'occlusion d'appendice auriculaire gauche selon la revendication 1, le dispositif d'occlusion d'appendice auriculaire gauche comprenant en outre au moins une barre d'ancrage (334) agencée sur la structure de cadre et faisant face à la plaque de fermeture (31).

6. Dispositif d'occlusion d'appendice auriculaire gauche selon la revendication 1, le cadre de fixation (33) comprenant en outre un film (356) qui est agencé sur la structure de cadre et recouvre au moins la surface circonférentielle de support.

7. Dispositif d'occlusion d'appendice auriculaire gauche selon la revendication 6, le cadre de fixation (33) comprenant en outre au moins une barre d'ancrage (334) qui est agencée sur la surface circonférentielle de support et fait face à la plaque de fermeture (31), et ladite au moins une barre d'ancrage pénétrant à travers le film (356).

8. Dispositif d'occlusion d'appendice auriculaire gauche selon la revendication 1, l'élément de raccordement (32) étant un élément de raccordement flexible ou un élément de raccordement élastique.

9. Dispositif d'occlusion d'appendice auriculaire gauche selon la revendication 8, l'élément de raccordement (32) comprenant une extrémité de raccordement proximale (321), une extrémité de raccordement distale (324), et un corps de raccordement (322) raccordé entre l'extrémité de raccordement proximale et l'extrémité de raccordement distale ;
l'extrémité de raccordement proximale (321) étant raccordée à la plaque de fermeture (31) ;
l'extrémité de raccordement distale (324) étant raccordée au cadre de fixation (33) ;
l'extrémité de raccordement distale présentant un coussinet sphérique (325) ; et
l'extrémité distale du corps de raccordement présentant une tête sphérique (323) adaptée au coussinet sphérique.

10. Dispositif d'occlusion d'appendice auriculaire gauche selon la revendication 9, le corps de raccordement (322) étant une tige élastique ou flexible ou une structure à ressort ou une structure tissée réalisée à partir d'une pluralité de filaments élastiques.

11. Dispositif d'occlusion d'appendice auriculaire gauche selon la revendication 1, la plaque de fermeture (31) étant une structure tissée à filaments à double couche.

12. Dispositif d'occlusion d'appendice auriculaire gauche selon la revendication 4, les tiges de support (334) formant un espace approximativement sphérique, chacune des tiges de support (334) comprenant une section en arc proximale (337), une section en arc distale (335), et une section en arc médiane (336) raccordée entre la section en arc proximale et la section en arc distale, et la section en arc proximale étant raccordée à un connecteur de fixation (331) ;
la section en arc proximale (337) faisant saillie vers l'extrémité distale du cadre de fixation (33) ;
la section en arc médiane (336) faisant saillie en éloignement de l'extrémité distale du cadre de fixation (33) ;
la section en arc proximale et la section en arc médiane formant une courbe approximativement en forme de S ; et
la section en arc distale (335) faisant saillie en éloignement d'un centre de l'espace approximativement sphérique.

13. Dispositif d'occlusion d'appendice auriculaire gauche selon la revendication 12, le cadre de fixation (33) comprenant en outre une branche (348) raccordée à deux tiges de support (334) adjacentes, et des noeuds de raccordement entre les branches et les tiges de support (334) étant agencés dans les tronçons médians et/ou les extrémités distales des sections en arc distales (335).

14. Dispositif d'occlusion d'appendice auriculaire gauche selon la revendication 13, deux branches (348) étant raccordées entre deux tiges de support (334) adjacentes ; et
les deux branches (348) étant inclinées par rapport aux tiges de support (334), et des sens d'extension des deux branches étant à peu près parallèles, une branche étant raccordée entre les sections en arc distales (335) de deux tiges de support (334) adjacentes, et une extrémité de l'autre branche étant raccordée au tronçon médian de la section en arc distale d'une tige de support, et l'autre extrémité de l'autre branche étant raccordée à l'extrémité distale de la section en arc distale.
